Europäisches Patentamt

European Patent Office

Office européen des brevets

(11) Publication number: **0 302 681**
**A2**

(12) **EUROPEAN PATENT APPLICATION**

(21) Application number: 88307063.3

(22) Date of filing: 01.08.88

(51) Int. Cl.⁴: **G 01 N 1/24**

(30) Priority: 07.08.87 GB 8718744

(43) Date of publication of application:
08.02.89 Bulletin 89/06

(84) Designated Contracting States:
AT BE CH DE ES FR GB GR IT LI LU NL SE

(71) Applicant: **LION TECHNOLOGY LIMITED**
**Ty Verlon Industrial Estate**
**Barry South Glamorgan CF6 3EQ (GB)**

(72) Inventor: **Williams, Paul Michael**
**The Cherries 2, Brookfield Avenue Cadoxton**
**Barry South Glamorgan (GB)**

(74) Representative: **Wynne-Jones, John Vaughan et al**
**Wynne-Jones, Lainé & James Morgan Arcade Chambers**
**33 St. Mary Street**
**Cardiff CF1 2AB (GB)**

(54) Fluid sampling systems.

(57) A gas sampling system particularly though not exclusively for alcohol breath testing, has a small suction pump including a diaphragm 16 connected by a passage 20 to one side 49 of a breath tube 10, the pump being arranged to draw the breath sample across the fuel cell 24. In addition there is a small bag 40 connected by the conduit 20 to a passage 48 on the other side of the breath tube. When the bag 40 expands it releases a latch 46 engaging a stud on a button 31 attached to the diaphragm 16. When the latch 46 is released the button is raised by the spring causing the diaphragm to draw in the breath sample.

FIG.1.

EP 0 302 681 A2

Bundesdruckerei Berlin

## Description

## Fluid Sampling Systems

This invention relates to sampling systems for extracting fluid samples from moving fluid passing through a confined flow passage. The invention is particularly though not exclusively applicable to a sampling system for use with an alcohol breath testing device. In many applications it is important to extract a fluid sample preferably of pre-determined volume and under controlled conditions which may involve a pre-determined time interval from a starting time. For example, in the case of alcohol breath testing it is important that a pre-determined volume of breath should be discarded before the sample is taken for the test, so that the test is made on true alveolar or deep-lung breath.

It has been proposed to provide a sampling system, for example, as described in British Patent No. 1448557 in which a subject is caused to blow down a breath tube and a small sample of breath is withdrawn from the tube and held in contact with an electro-mechanical "Fuel Cell", by means of a small positive displacement pump, including a piston actuated by a spring to draw in the sample and having a manual "set" button to compress the spring, and a latch which holds the piston with the spring in its stressed state. The latch can be released manually by a further manual button so that the sample is then drawn rapidly into contact with the fuel cell at the instant determined by the operator.

It has also been proposed in such a system to control the release of the latch automatically by an electrical system including a pressure sensing device responsive to the pressure of the fluid flow (i.e. the breath) in the flow passage, and coupled to an electronic timer. The combination of the pressure sensor and the timer is intended to provide a measure of the volume of breath flowing through the main passage so that a pre-determined discard volume is established before the sample of breath is extracted automatically. In practice however, such systems suffer from a number of disadvantages. For example, they may require a solenoid or equivalent electro-mechanical actuator and therefore demand a power supply of adequate size, which introduces further difficulties and disadvantages. A small replaceable dry cell battery would need to be changed repeatedly while a re-chargeable battery would be expensive and would also need to be recharged or replaced at short intervals. Existing electrical control systems also suffer from certain limitations in that they do not take account of the rate of flow of the breath through the main flow tube and only respond to an arbitrary basic pressure limit at a pre-determined set time interval. Accordingly it is an object of the invention to provide an improved fluid sampling system which will avoid some of the problems of existing systems and provide further novel advantages.

The invention from one aspect consists in apparatus for taking a fluid sample from a fluid stream along a flow passage, comprising a positive displacement pump or extraction system communicating with the flow passage and including a spring or other motor, a latch for holding the spring or motor in a pre-stressed or ready condition, and means for releasing the latch to cause the sample to be withdrawn, comprising an expansable chamber communicating with the same or a different but associated flow passage and having a movable wall or piston which is operatively connected to the latch.

The expansible chamber may contain a movable piston but is preferably a flexible sealed bag connected to the breath flow passage by a small bore duct or fluid channel. This provides a full sealed, fluid tight system and the parameters can be carefully designed and selected to provide the required conditions for the sampling.

According to a preferred feature of the invention when applied to alcohol breath testing, the extraction system is arranged to draw the sample into a chamber containing an electro-chemical cell arranged to generate an electrical signal in response to the presence of alcohol in the sample.

According to another preferred feature of the invention the fluid connection between the flow passage and the extraction system is separate from the communication between the flow passage and the latch release mechanism. For example, the flow passage may be effectively divided into two parallel passages, one communicating with the sample extraction system and the other with the latch release system. Also according to another preferred feature of the invention the divided fluid passage which communicates with the latch release system is effectively more restricted down-stream of the communicating passage so as to increase the relative pressure in the latch release system. For example, the flow passage may have an internal flow separating wall which is so arranged that one part has a relatively restricted inlet and a larger outlet and vice versa.

From another aspect the invention consists in a breathing tube for a breath sampling device of the type referred to, having two parallel flow passages, each with a separate individual lateral communicating port or passages. According to a preferred feature one of the two parallel flow passages is relatively restricted downstream of its lateral port or passage.

The invention may be performed in various ways and one specific embodiment will now be described by way of example with reference to the accompanying drawings, in which:-

    Figure 1 is a diagram illustrating the basic essentials of one example of a sampling system according to the present invention,

    Figure 2 is a diagrammatic sectional side elevation of a practical form of the invention,

    Figure 3 is a diagrammatic plan view of the example of Figure 2,

    Figures 4,5 and 6 are diagrammatic side elevations, illustrating the latching and trigger mechanism in different operative positions, and
Figure 7 is a side elevation of an alternative breathing tube.

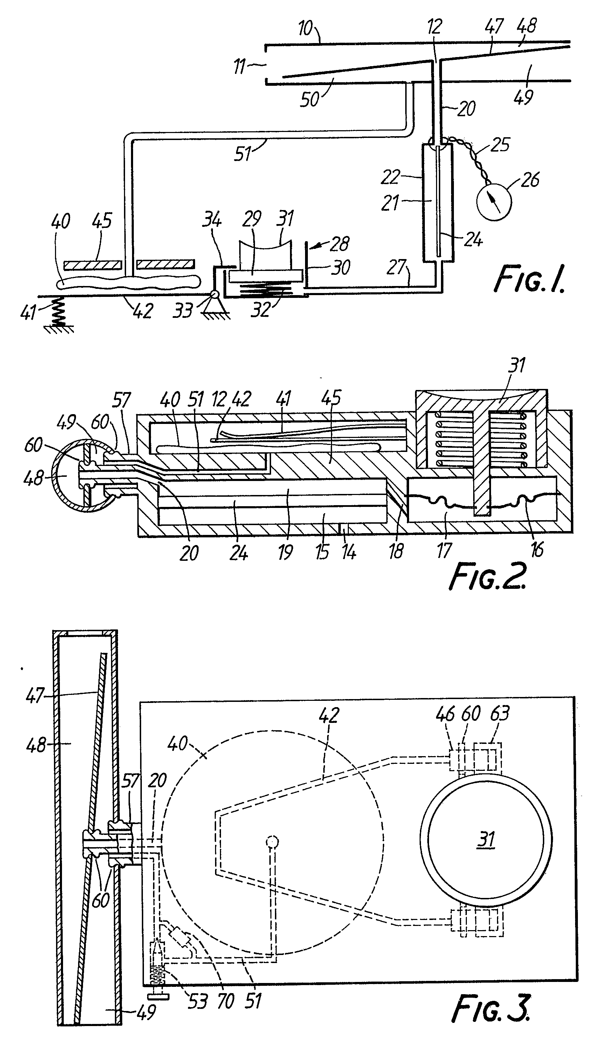

The alcohol breath tester and sampler illustrated diagrammatically in Figure 1 comprises a breathing tube 10, having a slightly constricted exit aperture 11 and a lateral opening 12, connected to a passage 20 which leads to a chamber 21 in a "fuel cell" unit 22 containing an electro-chemical fuel cell 24 of known type. The electrical output connections 25 from the cell lead to an electric display or indicating system 26, including a voltage detector and amplifier coupled to an audible or visual output, either analogue or digital as required, which provides a measure or indication of the alcohol content of the breath sample. The chamber 21 also communicates via a passage 27 with a positive displacement suction pump or withdrawal unit 28 including a piston or plunger 29 in a chamber 30. The plunger is attached to a manual press button 31, and is acted on by a spring 32 in a direction to draw gas inwards from the opening 12 across the fuel cell 24. When fully depressed the button and piston 29 are latched by a laterally moveable bell-crank lever 34 pivoted at 33 and having an arm 42 controlled by an inflatable bag 40 opposing a spring 41. When the bag 40 is inflated it reacts against a fixed wall 45, depresses the lever arm 42, and the latch 34 is withdrawn thus acting as a trigger whereby the piston 29 is urged upwards by the spring 32 to draw in the breath sample from the tube 10 via the opening 12.

The breath tube 10 has an internal lengthwise separating or dividing wall 47, which is inclined from end to end of the tube and forms two parallel flow passages 48,49. The upper passage 48 is open at both ends and from this passage leads the lateral conduit 12, 20 communicating with the fuel cell unit 24. The other passage 49 is progressively reduced in cross-section and constricted towards its exit end 50, so that the pressure therein is increased relative to that in the passage 48. This pressure is transmitted via conduit 51 to the flexible bag 40 and the cross section of the conduit and its length are so related to the volume of the bag and force of the spring 41 tending to contract the bag,and to the flow conditions within the passage 49, and the relative size of the passage 48, that the bag will only act through the lever 42 to release the latch-trigger 34 when an adequate breath discard volume has been passed through the tube 10. It will be noted that this automatic release or sampling system obviates the need for a set timer and an arbitrary breath pressure switch, and automatically compensates for variations in breath pressure by means of the closed volume bag 40.

In the practical embodiment of Figures 2 to 6, like parts are indicated by the same reference numerals and will not require full description. It will be noted that the flow passage 20, to the fuel cell 24, is positioned co-axially within a tube 57 leading from the sub-passage 49, and connected via conduit 51, to the flexible bag 40. This connection includes an adjustable control valve 53 in parallel with a non-return valve 70 arranged to provide an increased return flow out of the bag 40 back to the breath tube 10.Both the inner and outer tubes 20,57 are provided with quick release detachable connections 60,so that the complete tube 48,47 can be formed as an inexpensive disposable unit,to be replaced when needed by a fresh sterile unit.

The passage 20 leads to a space 19 above the fuel cell 20, and this space communicates via passage 18 with the space 17 below a diaphragm 16 attached to the stem of the press button 31, thus acting as the equivalent of piston 29 in Figure 1. The space 15 below the fuel cell is vented to atmosphere via openings 14.

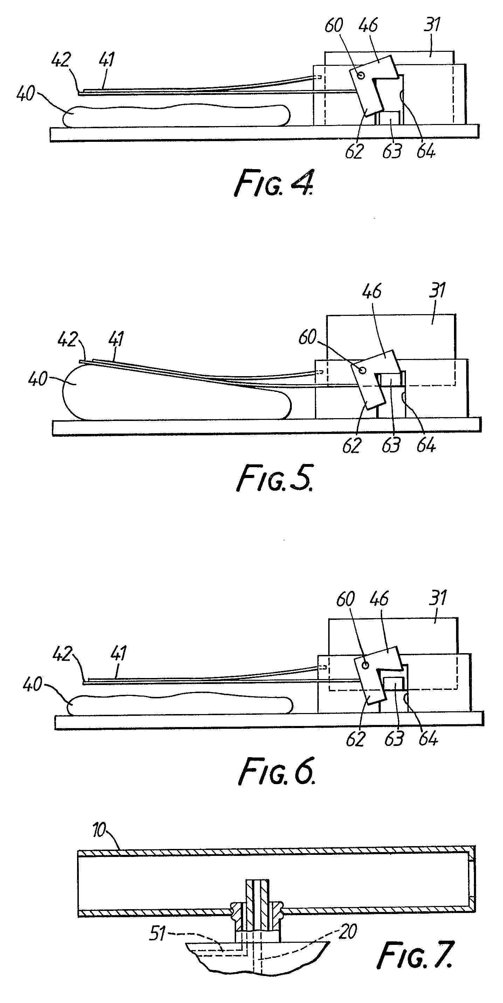

In this example the bag 40 is positioned above a fixed wall 45, and below a lever arm 12 and the spring 41. The lever arm 42 is looped see Figure 3,and the two arms of the loop are attached to a pair of latch trigger blocks 46 pivotally mounted on pins 60. Each trigger block is of L-shape with an upper horizontal limb 61 and a lower vertical limb 62. The latter cooperates with one of a pair of studs 63 attached to the button 31, and movable vertically in grooves 64. When the button rises or falls the latch operating sequence is illustrated in Figures 4 to 6.

In Figure 4 the bag 40 is collapsed, the lever 42 being urged downward onto the bag by the spring 41. Consequently the latch block 46 attached to the lever 42 is pivoted anti-clockwise into the position illustrated in which the lower limb 62 of the block engages above the stud 63 attached to the press button 31 thus holding the button and the diaphragm 16 in their lower positions.

When the subject blows through the tube 10 part of the breath enters the bag 40 through the passage 49 at a rate determined partly by the setting of the adjustable valve 53. When the bag reaches a predetermined volume,thus raising the lever 42 correspondingly against the pressure of the spring 41, the limb 46 of the latch block is shifted clear of the stud 63 and the press button 31 is accordingly released to move upwards under the influence of the spring 32. This raises the diaphragm 16 thus drawing a sample of breath from the passage 48 through the conduit 20, across the fuel cell 24 and through the passage 18 into the pumping chamber 17.

After the electronic circuit has produced an indication of the output from the fuel cell the whole unit can be reset by depressing the button 31, as illustrated in Figure 6. The two studs 63 on opposite sides of the button ride down the adjacent surfaces of the latch limbs 46 and cause the latch blocks to pivot slightly in a clockwise direction until the studs 63 are depressed below the lower ends of the limbs 46. The resilience of the lever 42 then causes the latch blocks to pivot into the position illustrated in Figure 4 where the studs 63 are latched downwards and the button 31 with the diaphragm 16 is also held downwards ready for the next operation. It will be noted that the release of the button and the suction pumping action of the diaphragm is effected automatically as a result of the breath pressure in the tube 10 without any manual operation.

In the examples described the breath tube 10 is divided by the wall 47 into the two flow passages 48,49 and a proportion of the breath is diverted from passage 49 into the bag 40 and another quantity is withdrawn from passage 48 across the fuel cell 24. It should be understood that there is no absolute necessity to provide dual flow passages 48,49. The breath sample for the fuel cell and the flow into the bag 40 could be derived from a single breath tube, as illustrated in Figure 7. Separate connections to separate passages are, however, preferred. In these examples the breath pressure energy in the tube 10 contributes to the extraction, avoids a latch solenoid and economises on electrical power requirements.

**Claims**

1. Apparatus for taking a fluid sample from a fluid stream along a flow passage, comprising a positive displacement pump or extraction system communicating with the flow passage and including a spring or other motor, a latch for holding the spring or motor in a pre-stressed or ready condition, and means for releasing the latch to cause the sample to be withdrawn, comprising an expansible chamber communicating with the same or a different but associated flow passage and having a movable wall or piston which is operatively connected to the latch.

2. Apparatus according to Claim 1 in which the expansable chamber is a flexible sealed bag connectd to the flow passage by a small bore duct or fluid channel.

3. Apparatus according to Claim 1 or Claim 2, for use in breath testing, in which the extraction system is arranged to draw the sample into a chamber containing an electro-chemical cell arranged to generate an electrical signal in response to the presence of a selected chemical substance in the breath sample.

4. Apparatus according to any of the preceding Claims in which the fluid connection between the flow passage and the extraction system is separate from the communication between the flow passage and the latch release mechanism.

5. Apparatus according to Claim 4 in which the flow passsage is effectively divided into two parallel passages, one communicating with the sample extraction system and the other with the latch release system.

6. Apparatus according to Claim 5 in which the divided flow passage which communicates with the latch release system, is effectively more restricted downstream of the communicating passage so as to increase the pressure in the latch release system.

7. Apparatus according to Claim 6 in which the flow passage has an internal flow separating wall which is so arranged that one part has a relatively restricted inlet and a larger outlet and vice versa.

8. A breathing tube for a breath sampling device of the type referred to, having two parallel flow passages, each with a separate individual lateral communicating port or passage.

9. A breathing tube according to Claim 8, in which one of the two parallel flow passages is relatively restricted downstream of its lateral port or passage.

10. Apparatus for taking a fluid sample from a flow stream along a flow passage comprising a positive displacement extraction system communicating with the flow passage and motor means for actuating the extraction system including an expansible chamber communicating with the flow passage and operatively associated with the extraction system whereby the pressure of the fluid stream contributes to the energy required to operate the extraction system.

0302681

FIG. I.

FIG. 2.

FIG. 3.

FIG. 4.

FIG. 5.

FIG. 6.

FIG. 7.